**Europäisches Patentamt**

**European Patent Office**

**Office européen des brevets**

(19)

(11) Veröffentlichungsnummer: **0 009 732**
**A1**

(12) # EUROPÄISCHE PATENTANMELDUNG

(21) Anmeldenummer: **79103571.0**

(22) Anmeldetag: **21.09.79**

(51) Int. Cl.³: **A 61 N 1/04**

(30) Priorität: **06.10.78 CH 10415/78**

(43) Veröffentlichungstag der Anmeldung:
**16.04.80 Patentblatt 80/8**

(84) Benannte Vertragsstaaten:
**BE CH DE FR GB NL**

(71) Anmelder: **Precimed S.A.**
**Chemin des Tourelles 17**
**CH-2400 Le Locle(CH)**

(72) Erfinder: **Babotai, Istvan, Dr.**
**Zürichholzstrasse 7**
**CH-8057 Zürich(CH)**

(74) Vertreter: **Buntz, Gerhard**
**Grenzacherstrasse 124 Postfach 601**
**CH-4002 Basel(CH)**

(54) **Katheter für Herzschrittmacher.**

(57) Katheter für Herzschrittmacher mit einer Stimulationselektrode.

Zur Erzielung einer sicheren und leichteren Postionierung der Stimulationselektrode und einer Sensorelektrode ist der Katheter in seinem vorderen Teil in einem dem Innendurchmesser des Ventrikels entsprechenden Abstand von der Skpitze des Katheters und auf einer dem Durchmesser des Vorhofs entsprechenden Länge in zwei oder mehr Zweige aufgespalten, die relativ zur Katheterachse einen nach aussen gerichteten Bogen bilden.

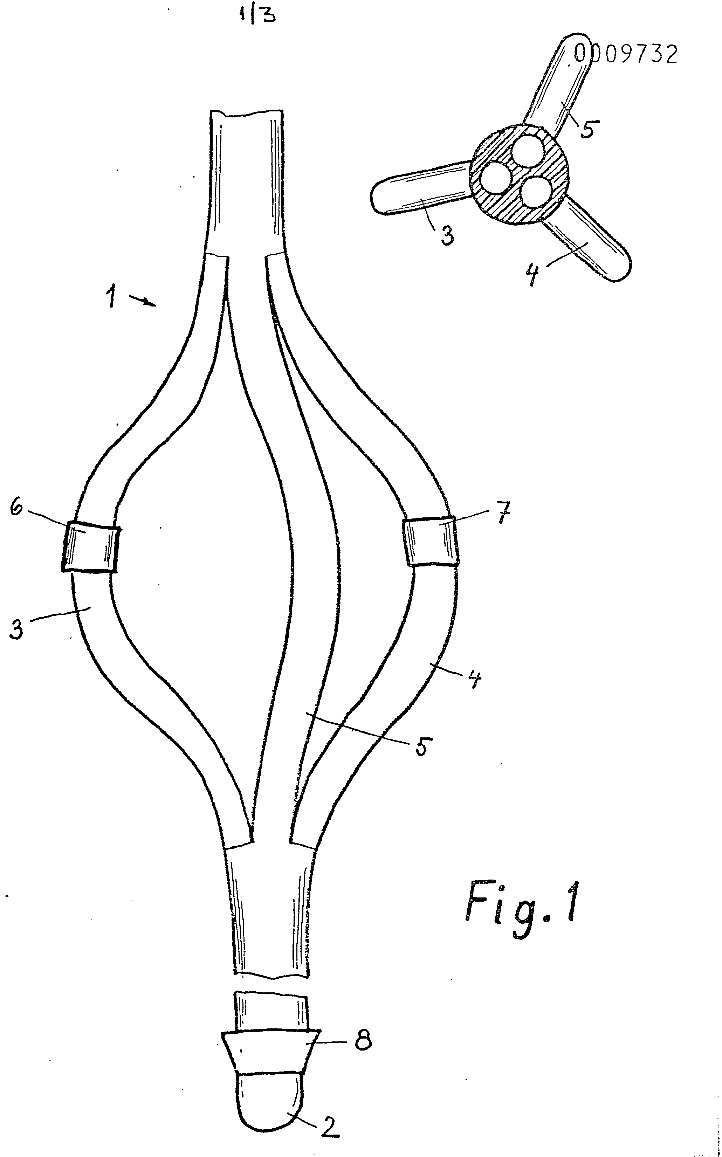

Fig. 1 zeigt die wesentlichen Merkmale dieses Katheters.

./...

Croydon Printing Company Ltd.

EP 0 009 732 A1

Fig.1

Ref. PD 4701/105

PRECIMED S.A., Le Locle

Katheter für Herzschrittmacher

Die Erfindung betrifft einen Katheter für Herzschrittmacher mit einer Stimulationselektrode.

Die Atriosynchrone-Stimulation ist die physiologisch
bestangepasste Methode, da bei ihr die ventrikuläre Depolarisation entsprechend der spontanen Vorhofaktivität gesteuert wird. Die Vorhofdepolarisation wird mit einer speziell konstruierten Sensorelektrode erfasst. Durch den gewonnenen Spannungsimpuls wird nach einem der normalen
atrioventrikulären Verzögerung entsprechenden Zeitintervall
der ventrikuläre Stimulationsimpuls ausgelöst und dem
Ventrikel über eine Stimulationselektrode zugeführt.
Dieses System hat gegenüber der üblichen nicht atriosynchronisierten Ventrikelstimulation den Vorteil einer
höheren Herzleistung und einer besseren Toleranz gegenüber

Bu/23.7.79

Anstrengungen. Allerdings hatten bisher bekannte Systeme auch einen Nachteil, der darin besteht, dass die zusätzliche Sensorelektrode im Vorhof schwierig zu positionieren ist.

Der Erfindung liegt daher die Aufgabe zu Grunde, einen Katheter bereitzustellen, mit dem diese vorteilhafte Stimulationsmethode durchgeführt werden kann, ohne dass die Probleme bei der Plazierung der Elektroden im Vorhof auftreten.

Erfindungsgemäss wird dies gelöst durch einen Katheter der eingangs genannten Art, der sich dadurch auszeichnet, dass er in seinem vorderen Teil in einem dem Innendurchmesser des Ventrikels entsprechenden Abstand von der Spitze des Katheters und auf einer dem Durchmesser des Vorhofs entsprechenden Länge eine Verweigung mit relativ zur Katheterachse bogenförmig nach aussen gerichteten Zweigen aufweist.

Zweckmässigerweise sind die Stimulationselektroden an der Spitze des Katheters und eine Sensorelektrode an wenigstens einem Zweig des Katheters angebracht.

In einer bevorzugten Ausführungsform sind mindestens drei Zweige vorgesehen, die zusätzlich spiralig verdreht sind.

In einer bevorzugten Ausführungsform sind die Zweige gegeneinander längsverschiebbar, mindestens ein Zweig gerade und ein anderer bogenförmig ausgebaucht.

Ein solcher Katheter hat gegenüber herkömmlichen Geräten für den gleichen Anwendungszweck erhebliche Vorteile. Wenn der Katheter in das Herz eingeführt ist, legen sich die nach aussen gebogenen Zweige an die Innenwand des Vorhofs, wodurch einerseits die Sensorelektrode ohne weiteres in Kontakt mit dem Gewebe gelangt und andererseits die ganze Anordnung gleichzeitig gegen Verschieben gesichert ist,

0009732

sodass sich die Stimulationselektrode nicht mehr leicht von ihrem Platz wegbewegen kann.

Durch eine 3- oder mehrfache Verzweigung und die Anbringung von mehr als einer Sensorelektrode lassen sich noch bessere Ergebnisse erzielen.

Nachfolgend werden anhand der beiliegenden Zeichnungen bevorzugte Ausführungsbeispiele der Erfindung beschrieben.

Es zeigen:

Figur 1 eine schematische Darstellung eines Stimulationskatheters mit einer dreifach-Verzweigung im Vorhofbereich.

Figur 2 einen ebenfalls dreifach verzweigten Katheter, der jedoch im Bereich der Verzweigung zusätzlich eine spiralige Verdrehung der Zweige aufweist.

Figur 3 eine andere Ausführungsform der Erfindung mit veränderlicher Verzweigung.

Figur 1 zeigt im wesentlichen den Teil eines Katheters, der sich in das innere des Herzens erstreckt. An seiner Spitze ist, wie üblich, eine Ventrikelelektrode angeordnet. In einem Abstand von der Spitze von etwa 5-15 cm ist der Katheter über eine Länge von etwa 4-6 cm in drei Zweige aufgespalten. Diese Zweige sind bogenförmig nach aussen ausgebaucht, sodass sich eine etwa korbähnliche Erweiterung des Katheters ergibt. Der Abstand zwischen der Spitze und dem vorderen Ende der Ausbauchung entspricht etwa dem Ventrikeldurchmesser, während die Länge, über die sich die Verzweigung erstreckt etwa dem Vorhofdurchmesser angepasst ist. Wegen der sehr unterschiedlichen Herzgrössen verschiedener Patienten ist es vorteilhaft verschiedene Abmessungen bzw. Kombinationen von Abmessungen vorzusehen.

0009732

Die Ausbauchung der Zweige wird durch eine permanente Vorspannung der metallischen Leiterspiralen im inneren der Zweige bewirkt. Die Ausbauchung ist etwa so stark, dass zwischen zwei Zweigen an der breitesten Stelle ein Abstand von ca. 6 cm entsteht.

An zwei Zweigen sind Sensorelektroden angebracht die zur Aufnahme der Atriumsignale dienen.

Unmittelbar hinter der Ventrikelelektrode ist eine der üblichen Vorrichtungen zur Verankerung der Elektrode im Gewebe vorgesehen.

Als Materialien für diese Katheterform können die üblichen bekannten Materialien verwendet werden. Die metallischen Leiterspiralen bestehen beispielsweise aus der mit Elgiloy bezeichneten Legierung, die mit Silikongummi umhüllt sind. ·

Zur Einführung wird bei diesem Katheter wie üblich ein Mandrin verwendet, der hier gleichzeitig dazu dient, die Verweigung geschlossen zu halten. Nach dem Entfernen des Mandrins nimmt die Verweigung die korbartige form an.

Ein Katheter der beschriebenen Art wird so plaziert, dass die Verzweigung sich im Vorhof ausdehnen kann und sich dadurch die Zweige dort an die Innenwand anlegen. Dadurch wird der Katheter in allen Richtungen fixiert und die Elektrode kann sich praktisch nicht mehr von der Kontaktstelle im Ventrikel entfernen.

Durch die an den Zweigen angebrachten Sensorelektroden wird die Vorhofdepolarisation erfasst und mit Hilfe des gewonnenen Signals die Ventrikelstimulation synchronisiert. Damit wird gleichzeitig mit einer optimalen Fixierung des Katheters eine nach dem heutigen Stand der Technik optimale Stimulationsmethode möglich.

0009732

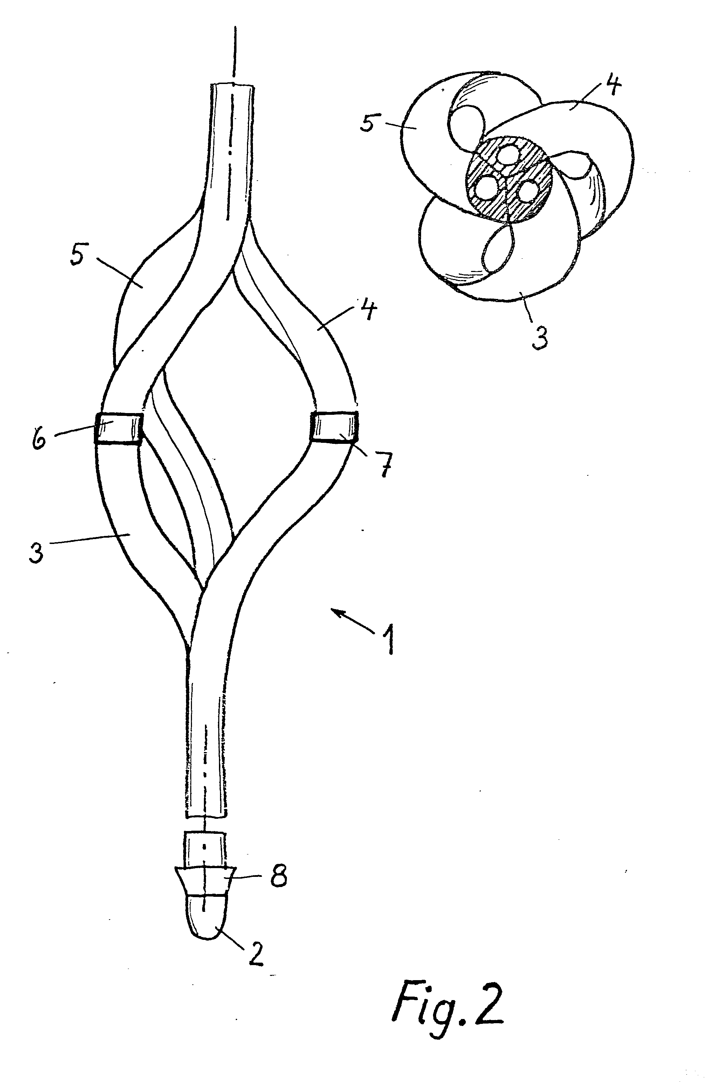

Die in Figur 2 gezeigte Ausführungsform hat gegen der gemäss Figur 1 noch den zusätzlichen Vorteil, dass sie sich besser an die Innenwand des Vorhofs anpasst und zwar sowohl an verschiedene Grössenverhältnisse als auch der Toleranz gegenüber Bewegungen. Dies wird dadurc erreicht, dass die einzelnen Zweige zusätzlich zur bogenförmigen Ausbauchung noch spiralförmig verdreht sind, wodurch sich eine Form ergibt, die den Rippen einer orientalischen Kuppel ähnlich ist. Eine solche Konfiguration hat bekanntlich den Vorteil, dass sie eine erhöhte Flexibilität und Anpassungsfähigkeit der einzelnen Zweige an bestehende Randbedingungen aufweist. In allen weiteren Merkmalen entspricht diese Ausführungsform der in Figur 1 gezeigten Version.

Die zu den beiden Figuren gehörenden Querschnittbilder zeigen zwei verschiedene Versionen des Katheterquerschnitts. Der in Figur 1 gezeigte Querschnitt ist besonders einfach, hat aber den Nachteil eines insgesamt grösseren Durchmessers des Katheters im nichtverzweigten Bereich. Der in Figur 2 gezeigte Querschnitt zeichnet sich dadurch aus, dass die einzelnen Zweige den Querschnitt von Kreissektoren besitzen und dadurch ein kleinerer Durchmesser im nichtverzweigten Bereich erzielt werden kann.

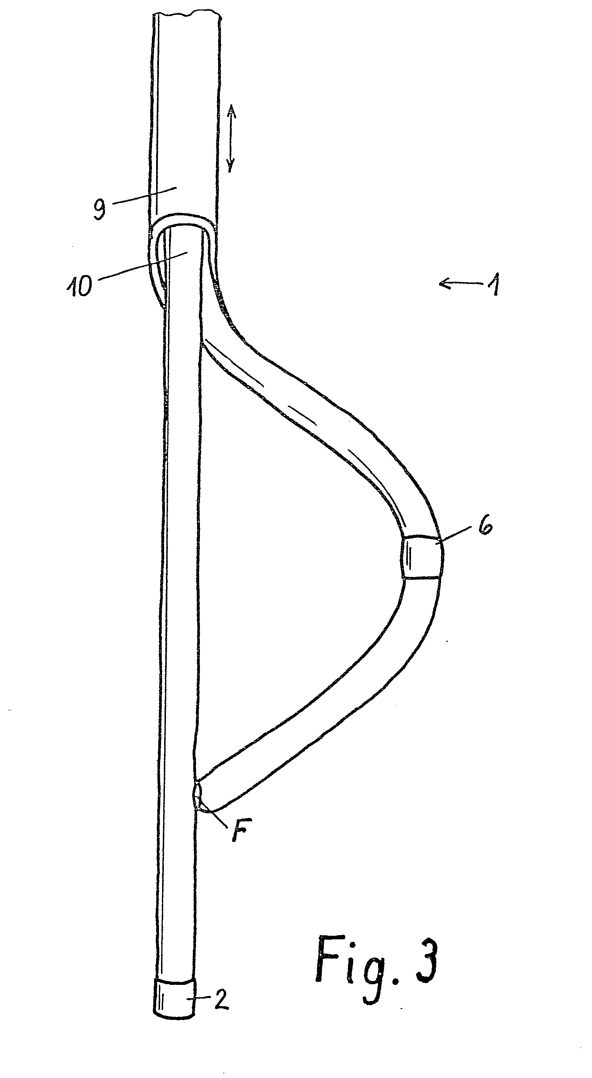

Bei der in Figur 3 gezeigten Version besteht der gesamte Katheter aus zwei konzentrisch ineinander verlaufenden Teilen, die sich ebenfalls im Bereich des Verhofs verzweigen und an ihrem Ende fest miteinander verbunden sind. Dadurch, dass die beiden (oder mehr) Zweige zu getrennten Katheterteilen gehören, lassen sie sich gegeneinander verschieben, was zu einer Veränderung des Querdurchmessers der Verweigung führt. Auf diese Weise kann die Verzweigung besser den anatomischen Gegebenheiten angepasst werden. Der Verbindungspunkt F kann ausserdem verschiebbar am geraden Teil angebracht sein.

Das Prinzip der vorliegenden Erfindung kann selbstverständlich auch für einen Katheter benutzt werden, mit dem

- 6 -

0009732

lediglich eine Ventrikelstimulation durchgeführt werden soll, ohne eine Atriumsynchronisation vorzusehen. In diesem Fall kann auf die Sensorelektroden an den Zweigen verzichtet werden. Umgekehrt ist es auch möglich, mit einem Katheter nach der Erfindung eine Atriumstimulation durchzuführen. Für diesen Fall ist mindestens eine der an den Zweigen angebrachten Elektroden eine Stimulationselektrode.

00.09732

Patentansprüche

1. Katheter für Herzschrittmacher mit einer Stimulationselektrode, dadurch gekennzeichnet, dass er in seinem vorderen Teil in einem dem Innendurchmesser des Ventrikels entsprechenden Abstand von der Spitze des Katheters und auf einer dem Durchmesser des Vorhofs entsprechenden Länge eine Verweigung mit relativ zur Katheterachse bogenförmig nach aussen gerichteten Zweigen aufweist.

2. Katheter nach Anspruch 1, dadurch gekennzeichnet, dass an wenigstens einem Zweig eine Sensorelektrode angebracht ist.

3. Katheter nach Anspruch 1, dadurch gekennzeichnet, dass die Stimulationselektrode an der Spitze des Katheters angebracht ist.

4. Katheter nach Anspruch 1, dadurch gekennzeichnet, dass die Stimulationselektrode an einem Zweig angebracht ist.

5. Katheter nach Anspruch 1, dadurch gekennzeichnet, dass die einzelnen Zweige spiralförmig verdreht sind.

6. Katheter nach Anspruch 1, dadurch gekennzeichnet, dass die einzelnen Zweige den Querschnitt von Kreissektoren besitzen.

7. Katheter nach Anspruch 1, dadurch gekennzeichnet, dass die Zweige gegeneinander längsverschiebbar sind, mindestens ein Zweig gerade und mindestens im weiterer bogenförmig ausgebaucht ist.

* * *

0009732

1

6

3

5

7

4

5

8

2

*Fig.1*

.0009732

Fig.2

.0009732

9

10

← 1

6

F

2

# Fig. 3

# EUROPÄISCHER RECHERCHENBERICHT

## EINSCHLÄGIGE DOKUMENTE

| Kategorie | Kennzeichnung des Dokuments mit Angabe, soweit erforderlich, der maßgeblichen Teile | betrifft Anspruch |
|---|---|---|
| | US - A - 3 890 977 (WILSON)<br>* Spalte 3, Zeile 62 - Spalte 4, Zeile 3; Figuren 3a,3b * | 1,2 |
| | -- | |
| | US - A - 4 057 067 (LAJOS)<br>* Spalte 2, Zeilen 54-64; Figur 3; Spalte 3, Zeilen 35-45 * | 1-4 |
| | -- | |
| P | US - A - 4 154 247 (MEDTRONIC)<br>* Spalte 6, Zeile 55 - Spalte 7, Zeile 45; Figuren 4a-4g,5 * | 1-4,7 |
| | ---- | |

**KLASSIFIKATION DER ANMELDUNG (Int.Cl. 3)**

A 61 N 1/04

**RECHERCHIERTE SACHGEBIETE (Int. Cl. 3)**

A 61 N 1/04

**KATEGORIE DER GENANNTEN DOKUMENTE**

X: von besonderer Bedeutung

A: technologischer Hintergrund

O: nichtschriftliche Offenbarung

P: Zwischenliteratur

T: der Erfindung zugrunde liegende Theorien oder Grundsätze

E: kollidierende Anmeldung

D: in der Anmeldung angeführtes Dokument

L: aus andern Gründen angeführtes Dokument

&: Mitglied der gleichen Patentfamille, übereinstimmendes Dokument

Der vorliegende Recherchenbericht wurde für alle Patentansprüche erstellt.

| Recherchenort | Abschlußdatum der Recherche | Prüfer |
|---|---|---|
| Den Haag | 17-01-1980 | BIJN |

EPA form 1503.1   06.78